**Europäisches Patentamt**

**European Patent Office**

**Office européen des brevets**

(19)

(11) **EP 1 217 292 A1**

(12) **EUROPÄISCHE PATENTANMELDUNG**

(43) Veröffentlichungstag:
26.06.2002 Patentblatt 2002/26

(51) Int Cl.⁷: **F21V 33/00**, H04N 5/225

(21) Anmeldenummer: 01126069.2

(22) Anmeldetag: 02.11.2001

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **19.12.2000 DE 10063380**

(71) Anmelder: **Heraeus Med GmbH**
**D-63450 Hanau (DE)**

(72) Erfinder:
• **Keitel, Joachim**
**63486 Bruchköbel (DE)**

• **Gampe, Uwe**
**47137 Duisburg (DE)**
• **Hartge, Jörg Eduard, Dr.**
**63571 Gelnhausen (DE)**
• **Helten, Matthias**
**34320 Kassel (DE)**
• **Marka, Rudolf, Dr.**
**60320 Frankfurt (DE)**

(74) Vertreter: **Kühn, Hans-Christian**
**Heraeus Holding GmbH,**
**Schutzrechte,**
**Heraeusstrasse 12-14**
**63450 Hanau (DE)**

(54) **Verfahren und Vorrichtung zur Video-Aufnahme eines beleuchteten Feldes**

(57) Zur Video-Aufnahme eines mittels einer Leuchte, insbesondere Operationsleuchte, beleuchteten Feldes, wobei aus wenigstens einem Lichtaustrittsbereich eines Leuchtengehäuses wenigstens ein Lichtbündel zur Bestrahlung des Feldes in Richtung einer vorgegebenen Gehäuse-Achse austritt, wird eine im Abstand zum Lichtaustritt des Lichtbündels schwenkbar angeordnete Video-Kamera mit ihrer optischen Achse mittels eines Stellgliedes selbsttätig so lange verstellt, bis sie das wenigstens eine Lichtbündel im Bereich des beleuchteten Feldes schneidet. Das Stellglied wird dabei durch Signale aus einer Steuereinheit angesteuert. Vorzugsweise wird die Video-Kamera schrittweise verstellt. Dabei wird durch Scharfeinstellung mit Hilfe einer Autofokus-Funktion der Video-Kamera der Abstand zum beleuchteten Feld ermittelt und anschließend mittels der Steuereinheit Signale an das Stellglied der Kamera zum Ausgleich einer Parallaxe zwischen der optischen Achse der Kamera und des bzw. der aus der Leuchte austretenden Lichtbündel ausgegeben.

EP 1 217 292 A1

**Beschreibung**

Die Erfindung betrifft ein Verfahren zur Video-Aufnahme eines mittels einer Leuchte, insbesondere Operationsleuchte, beleuchteten Feldes, wobei aus wenigstens einem Lichtaustrittsbereich eines Leuchtengehäuses wenigstens ein Lichtbündel zur Bestrahlung des Feldes in Richtung einer vorgegebenen Gehäuse-Achse austritt, sowie eine Vorrichtung.

Aus der DE 195 23 377 C1 ist ein Verfahren zur Übertragung von Video-Signalen sowie ein Bildübertragungssystem für Operationsleuchten bekannt. Hierzu ist eine Operationsleuchte mit einer Video-Kamera im Bereich des Leuchtengehäuses zur externen bildlichen Darstellung des Operationsfeldes mittels einer Bildschirm/Kontrollvorrichtung vorgesehen, wobei das Leuchtengehäuse über eine verstellbare Aufhängung mit einer Aufhängekonsole im Deckenbereich eines Operationssaales verbunden ist.

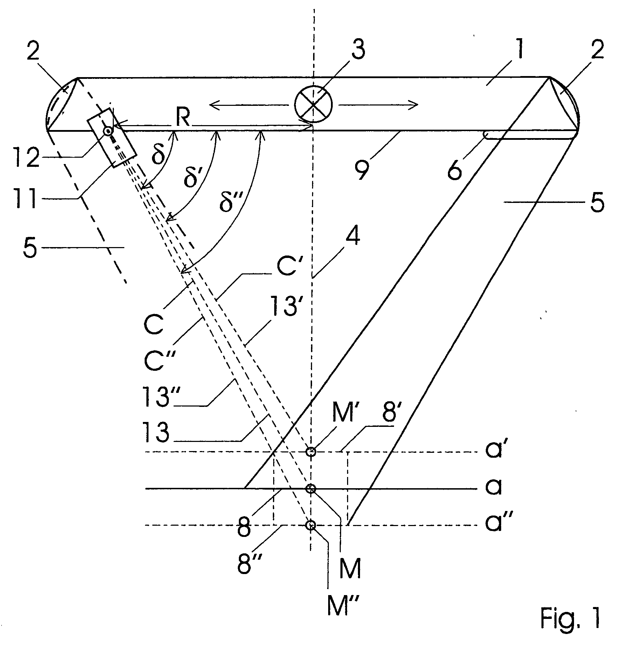

In Figur 1 der DE-Schrift wird eine Leuchte gezeigt, bei der die Video-Kamera außermittig angebracht ist.

Als problematisch erweist sich bei einer solchen Einrichtung, dass eine Verstellung der Operationsleuchte relativ zum Aufnahmeobjekt bzw. Operationsgebiet auch eine Verstellung der Orientierung des Bildes zum Operationsfeld bzw. eine Änderung des Bildausschnitts zur Folge hat; hierdurch erscheint dem Operateur unter Umständen eine winkelmäßige Verschiebung bzw. Verdrehung zwischen Operationsfeld und Bildwiedergabe auf dem Monitor.

Weiterhin ist aus der EP 0 989 744 A2 eine Bildübertragungseinrichtung für eine Video-Kamera in Verbindung mit einer Operationsleuchte und einer Kontrollvorrichtung mit Bildschirm und Steuereinrichtung bekannt, die wenigstens einen Bildsensor der Kamera in einer verstellbaren Lagerung aufweist, wobei wenigstens eine mittels der Steuereinrichtung betätigte Antriebsvorrichtung zur Positionierung wenigstens des Bildsensors relativ zum Aufnahmeobjekt vorgesehen ist; die Video-Kamera ist dabei mit einem Leuchtenkörper einer Operationsleuchte verbunden, die mittels verstellbarer Aufhängung in einer stationären Befestigung gehalten ist, wobei zumindest der Bildsensor der Video-Kamera um deren optische Achse drehbar gelagert ist; die Video-Kamera ist in einem im Leuchtenkörper der Operationsleuchte befindlichen Drehlager angeordnet, wobei die Achse des Drehlagers wenigstens parallel zur optischen Achse der Video-Kamera verläuft.

Als problematisch erweist es sich, dass hier nur eine Frontaldarstellung entlang der Richtung der Beleuchtung des Aufnahmeobjektes möglich ist.

Wenn die Kamera, insbesondere Video-Kamera, an einer Leuchte, beispielsweise einer Operationsleuchte, befestigt werden soll, kann es notwendig oder gewünscht sein, die Kamera außerhalb der optischen Achse dieser Leuchte anzuordnen. In dem Fall muss die optische Achse der Kamera in einem Winkel zur optischen Achse der Leuchte stehen, um auf den Mittelpunkt des beleuchteten Feldes gerichtet zu sein. Man spricht in einem solchen Fall von einer Parallaxe zwischen beiden Objekten.

Aufgabe der Erfindung ist es, die Parallaxe bei Verstellung der Leuchte gegenüber dem beleuchteten Gegenstand so zu korrigieren, dass die optische Achse der Kamera - bzw. Video-Kamera - auch nach der Verstellung wieder auf dem Mittelpunkt des beleuchteten Feldes ausgerichtet ist.

Die Aufgabe wird verfahrensgemäß dadurch gelöst, dass eine im Abstand zum Lichtaustrittsbereich des Lichtbündels verstellbar bzw. schwenkbar angeordnete Video-Kamera mit ihrer optischen Achse mittels eines Stellgliedes selbsttätig so lange verstellt wird, bis sie das wenigstens eine Lichtbündel im Bereich des beleuchteten Feldes schneidet.

Als vorteilhaft erweist es sich, dass der beleuchtete Gegenstand mit unterschiedlichen Perspektiven betrachtet werden kann, so dass auch entfernte Beobachter sich ein passendes Bild bzw. eine räumliche Vorstellung am zugehörigen Monitor machen können.

Das Stellglied wird vorzugsweise durch Signale aus einer Steuereinheit angesteuert.

In einer bevorzugten Ausgestaltung des Verfahrens wird die Video-Kamera relativ zur vorgegebenen Gehäuse-Achse schrittweise so lange verstellt, bis die optische Achse der Kamera das Lichtbündel bzw. die vorgegebene (Gehäuse-)Achse wenigstens näherungsweise schneidet.

Vorzugsweise wird die Video-Kamera schrittweise verstellt. Durch Scharfeinstellung mit Hilfe einer Autofokus-Funktion der Video-Kamera wird der Abstand zum beleuchteten Feld ermittelt und anschließend mittels der Steuereinheit Signale an das Stellglied der Kamera zum Ausgleich einer Parallaxe zwischen der optischen Achse der Kamera und des bzw. der Lichtbündel ausgegeben.

Es erweist sich als vorteilhaft, das Bild vor dem Scharfstellen zu vergrößern. Dazu wird vor dem Aktivieren der Scharfstellungsfunktion, also beispielsweise der Autofokusfunktion der Kamera, die Brennweite des Zoom-Objektivs maximal vergrößert ("Tele"-Stellung). Nachdem das Bild scharf gestellt ist, wird die Brennweite wieder auf den ursprünglichen Wert zurück gebracht.

Vorteilhafterweise erfolgt eine Korrektur der Ausrichtung der optischen Achse der Kamera auf iterativem Wege.

Vorteilhafte Ausgestaltungen des Verfahrens sind in den Ansprüchen 2 bis 8 angegeben.

Die Aufgabe wird für eine Leuchte, insbesondere Operationsleuchte, mit wenigstens einem Leuchtgehäuse aus dem wenigstens ein Lichtbündel zur Bestrahlung eines beleuchteten Feldes in Richtung einer vorgegebenen Achse austritt, wobei das Leuchtungsgehäuse wenigstens eine Video-Kamera aufweist, die im Abstand zum Austrittsbereich des Lichtbündels oder der Lichtbündel bzw. der vorgegebenen Achse angeordnet ist, dadurch gelöst, dass die Video-Kamera mit ihrer optischen Achse gegenüber dem wenigstens einen Lichtbündel bzw. gegenüber der vorgegebenen Achse mittels eines Stellgliedes durch Stellsignale aus einer Steuervorrichtung selbsttätig so weit verstellbar ist, dass die optische Achse der Kamera das beleuchtete Feld schneidet.

Als besonders vorteilhaft erweist sich eine rasche Nachführung der Kamera, die praktisch automatisch erfolgt.

In einer bevorzugten Ausgestaltung der Leuchte ist zur Ansteuerung des Stellgliedes der Video-Kamera eine Steuer-Einheit bzw. ein Nachlauf-Regler vorgesehen, welcher Führungs-Signale erhält, die von der Ausrichtung der vorgegebenen Achse des Leuchtengehäuses und dem von der Kamera gemessenen Abstand zum beleuchteten Feld abhängig ist.

Vorteilhafterweise erfolgt die Korrektur des Abstandes zum beleuchteten Feld auf iterativem Wege, wobei die Steuereinheit über die Kameraausrichtung anhand der Zahl der vorgenommenen Verstellschritte und über die Entfernung zum beleuchteten Feld durch die Autofokus-Funktion der Kamera informiert. Hieraus lässt sich anhand einer trigonometrischen Umformung auf einfache Weise der Abstand zwischen dem Leuchtengehäuse und dem Beleuchtungsfeld ermitteln, wobei der Abstand zwischen der Achse des Leuchtengehäuses und der Video-Kamera stets konstant bleibt.

Zur Bildung des jeweiligen Führungs-Signals kann auch ein Winkel-Sensor eingesetzt werden, der eine räumliche Ausrichtung der optischen Achse der Kamera erfasst.

Vorteilhafte Ausgestaltungen der Leuchte sind in den Ansprüchen 10 bis 12 angegeben.

Im folgenden ist der Gegenstand der Erfindung anhand der Figuren näher erläutert.

Figur 1 zeigt einen Längsschnitt durch eine erfindungsgemäße Operationsleuchte, wobei die Leuchte und der Strahlengang schematisch dargestellt sind.

Figur 2 zeigt als Blockschaltbild ein Flussdiagramm zur Folgeregelung der Video-Kamera mit ihrer optischen Achse.

Gemäß Figur 1 weist das Leuchtengehäuse 1 der Operationsleuchte einen hier schematisch dargestellten Ringreflektor 2 auf, welcher von wenigstens einer - in Längsachse 4 - zentral angeordneten Lichtquelle 3 in der schematisch dargestellten Position bestrahlt wird. Der Ringreflektor 2 ist dabei so ausgebildet, dass sich ein axialsymmetrisches bzw. konzentrisches Strahlenbündel 5 (zwecks besserer Übersicht nur im Teilausschnitt gezeigt) entlang der Längsachse 4 bildet, welches auf dem beleuchteten Feld 8 für eine schlagschattenfreie Beleuchtung sorgt. Auf diese Weise ist es möglich, dass in das Lichtbündel 5 eventuell ragende Gegenstände, wie z.B. Instrumente des Operateurs, bzw. Hände oder Kopf des Chirurgen keine Schlagschatten werfen und somit auch das Operationsfeld stets gut beleuchtet bleibt.

Auf der zum beleuchteten Feld 8 gerichteten unteren Seite 9 des Leuchtengehäuses 1 ist exzentrisch zu der Längsachse 4 im Abstand eine Video-Kamera 11 angeordnet, welche eine perspektivische Darstellung des Operationsfeldes - beispielsweise einer Wunde - dem Betrachter am Bildschirm ermöglicht. Die Video-Kamera 11 ist in einer Halterung 12 gelenkig angeordnet, so dass deren optischen Achse 13 bzw. der Schwenkwinkel δ der Kamera (Winkel zwischen der optischen Achse 13 und einer senkrecht zur Achse 4 verlaufenden Ebene bzw. Lichtausschnittsfläche) mit Hilfe eines hier nicht dargestellten Stellgliedes so eingestellt werden kann, dass die optische Achse 13 auf den Mittelpunkt M des beleuchteten Feldes 8 gerichtet ist und somit zumindest näherungsweise die Längsachse 4 in einem kleinen Umkreis schneidet. Sobald nun der Abstand a zwischen Leuchtengehäuse 1 bzw. Lichtaustrittsfläche 6 und beleuchteten Feld 8 von a auf a' verringert wird, muss die optische Achse 13 der Kamera stärker konvergieren, um noch das neue Zentrum M' des beleuchteten Feldes 8' erfassen zu können. Die Neuausrichtung der optischen Achse bei Verkürzung auf Abstand a' ist mit 13' bezeichnet.

Da die Entfernung C zwischen Kamera 11 und beleuchteten Feld 8 sich nunmehr auf einen Wert C' verkürzt, ist es aufgrund trogonometrischer Berechnung ($\cos\delta = R/C$ bzw. $\cos\delta' = R/C'$) möglich, mittels Arcusfunktion den Winkel δ' zu ermitteln, woraus sich auch der Abstand a ($a = c.\sin\delta$ bzw. $a' = c'.\sin\delta'$) ergibt

Aufgrund des neu errechneten Winkels δ' wird die Kamera mit Hilfe einer hier nicht dargestellten Steuereinheit und eines als Stellglied wirkenden Antriebsmotors schrittweise so lange verschwenkt bis der errechnete Wert für δ' erreicht ist.

Umgekehrt wird bei einer Vergrößerung des Abstandes zwischen den hier schematisch dargestellten Lichtaustrittsflächen 6 des Leuchtengehäuses 1 und dem beleuchteten Feld 8" auf a" ein steilerer Winkel der optischen Achse 13" eingestellt werden, damit die. Video-Kamera 11 weiterhin das Zentrum des beleuchteten Feldes 8" anvisieren kann. Die entsprechenden trigonomenischen Beziehungen lauten dann:

$$\cos\delta'' = R/C'' \text{ bzw. } a'' = c'' . \sin\delta''$$

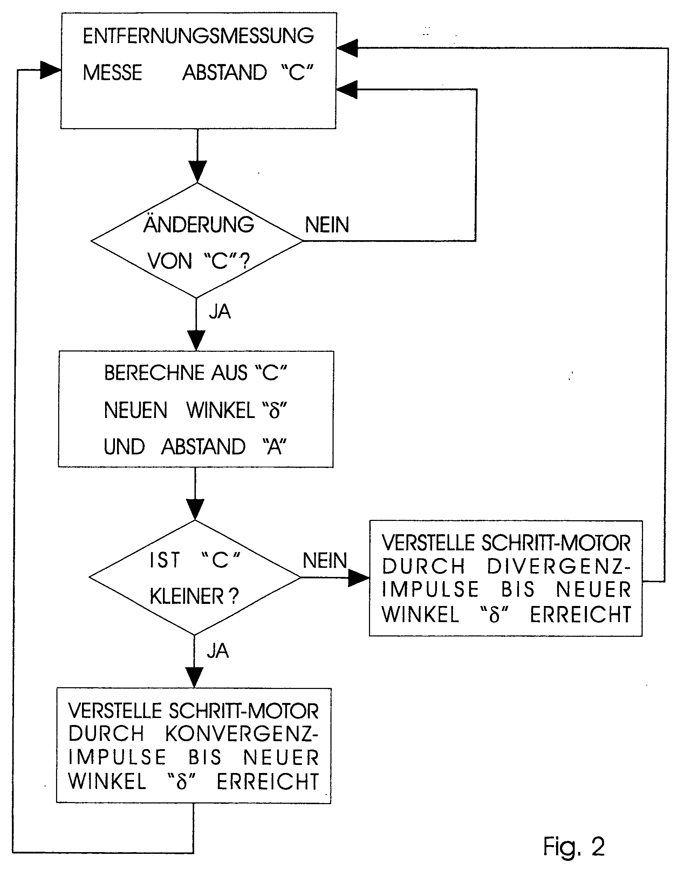

Mit Hilfe einer - zwecks besserer Übersicht hier nicht gezeigten - Steuereinheit (digitale Recheneinheit) wird in einer ersten Ausführungsform gemäß Figur 2 die Parallaxe schrittweise folgendermaßen korrigiert:

1. Entfernung messen
2. Winkel entsprechend der Entfernung einstellen
3. Entfernung messen
4. Falls Ergebnis 3 ungleich Ergebnis 1, weiter bei 1

Prinzipiell kann davon ausgegangen werden, dass dieses Verfahren auch in ungünstigen Fällen nach einigen (maximal vier oder fünf) Schnitten stabil ist. Um Fehler abzufangen, wurde die Steuereinheit so programmiert, dass dann, wenn das Verfahren sich nicht stabilisiert, der Winkel entsprechend einem Standard-Abstand eingestellt wird (bei Operationsleuchten beträgt dieser Abstand ein Meter).

Weiterhin wird mit Hilfe der Steuereinheit in einer zweiten Ausführungsform die Parallaxe schrittweise folgendermaßen korrigiert:

1. Beleuchtungsstärke messen
2. Kamera eine Stufe nach <u>außen</u> kippen
3. Beleuchtungsstärke messen
4. Wenn Ergebnis 3 größer Ergebnis 1, dann weiter bei 1, sonst weiter bei 5
5. Beleuchtungsstärke messen
6. Kamera eine Stufe nach <u>innen</u> kippen
7. Beleuchtungsstärke messen
8. Wenn Ergebnis 7 größer Ergebnis 5, dann weiter bei 5

In dieser Ausführungsform wird vorzugsweise das bei Video-Kameras (für Operationsleuchten) ohnehin vorhandene Helligkeits-Signal als Teil der automatischen Beleuchtungseinstellung zur Ermittlung der Beleuchtungsstärke verwendet.

Alternativ kann die Beleuchtungsstärke auch mit einem in der bzw. an der Video-Kamera angeordneten Belichtungsmesser ermittelt werden.

Als besonders vorteilhaft erweist es sich, dass Sensor bzw. Sensoren und Stellelemente sich in der Video-Kamera 11 befinden, bzw. mit ihrer Halterung 12 verbunden sind, so dass hier eine kompakte Regelung bzw. Ansteuerung unter guten Hygienebedingungen erfolgen kann.

Dabei wird mit Hilfe einer in der Kamera 11 eingebauten Autofokussierung die Entfernung zwischen dem beleuchteten Feld 8 und der Kamera 11 ermittelt und anschließend eine Korrektur der Parallaxe soweit vorgenommen, dass aufgrund der bekannten Entfernung zwischen Kamera und beleuchteter Ebene ein Parallaxausgleich erfolgen kann. Dies geschieht vorzugsweise auf iterativem Wege, wie es nachfolgend an einem Beispiel erläutert wird:

In ihrer ursprünglichen Aufgabenstellung weist die Leuchte beispielsweise eine Entfernung zur Wunde von 1,3 m auf und wird dann beispielsweise auf 0,8 m abgesenkt. Dann muss die Parallaxeneinstellung neu bestimmt und eingestellt werden. In der Praxis kommt es vor, dass der Leuchtenkörper schräg zum OP-Tisch gestellt wurde. Dann misst die Kamera in der ursprünglichen, für 1,4 m korrekten Parallaxe eine andere Entfernung, als sie für die neue Achse der Leuchte korrekt ist, denn durch die Schrägstellung ist die Entfernung im Peripheriebereich des Leuchtenkörpers (d.h. weiter außen) anders als in der Mitte. Nach der ersten Parallaxenkorrektur wird die Kamera damit näher an der Achse der Leuchte positioniert, befindet sich aber noch nicht in der korrekten Stellung. Außerdem wird das Bild etwas unscharf sein, da die Scharfstellung ja vor der Kippung erfolgt war.

Deshalb ist es dann, wenn die Leuchte nicht frontal auf den zu filmenden Gegenstand gerichtet ist, notwendig, die Parallaxenkorrektur einige Male zu wiederholen, bis sowohl die Entfernungsmessung als auch die Winkelstellung stabil sind. Dies bedeutet, dass zunächst bei Verstellung des Leuchtengehäuses 1 die Video-Kamera 11 mit ihrer optischen Achse so verschwenkt wird, dass sich die optische Achse 13 und die Längsachse 4 wenigstens näherungsweise schneiden. Durch diese schrittweise Vergrößerung des konvergierenden Winkels zwischen beiden Achsen gelangt die optische Achse 13 in eine Schnittebene mit dem Beleuchtungsfeld 8, woraufhin dann eine Entfernungsmessung mit Hilfe der Autofokuseinrichtung von Video-Kamera 11 erfolgt. Nachdem nun sowohl der Neigungswinkel der optischen Achse 13 zur Längsachse 4, als auch die Entfernung zwischen dem beleuchteten Feld 8 und der Video-Kamera 11 bekannt ist, erfolgt mittels - eines vorzugsweise digitalen - Steuergeräts eine Berechnung der trigonometrischen Verhältnisse, woraufhin anschließend die Video-Kamera 11 mit ihrer optischen Achse 13 so verstellt wird, dass sie die Längsachse 4 des Leuchtengehäuses 1 näherungsweise im Zentrum des beleuchteten Feldes 8 schneidet und somit Parallaxenfreiheit bei der Bildaufnahme besteht.

**Patentansprüche**

1. Verfahren zur Video-Aufnahme eines mittels einer Leuchte, insbesondere Operationsleuchte, beleuchteten Feldes, wobei aus wenigstens einem Lichtaustrittsbereich eines Leuchtengehäuses wenigstens ein Lichtbündel zur Bestrahlung des Feldes in Richtung einer vorgegebenen (Gehäuse-) Achse austritt, **dadurch gekennzeichnet, dass** eine im Abstand zum Lichtaustritt des Lichtbündels schwenkbar angeordnete Video-Kamera mit ihrer optischen Achse mittels eines Stellgliedes selbsttätig so lange verstellt wird, bis sie das wenigstens eine Lichtbündel im Bereich des beleuchteten Feldes schneidet.

2. Verfahren nach Anspruch 1, **dadurch gekennzeichnet, dass** das Stellglied durch Signale aus einer Steuereinheit angesteuert wird.

3. Verfahren nach Anspruch 1 oder 2, **dadurch gekennzeichnet, dass** die Video-Kamera relativ zur vorgegebenen Gehäuse-Achse so lange verstellt wird, bis die optische Achse der Kamera die vorgegebene (Gehäuse-)Achse wenigstens näherungsweise schneidet.

4. Verfahren nach einem der Ansprüche 1 bis 3, **da-**

**durch gekennzeichnet, dass** der nötige Verstellweg dadurch ermittelt wird, dass die Kamera zunächst den gesamten Stellbereich durchfährt und dann die Position der höchsten Bildhelligkeit einnimmt.

5. Verfahren nach einem der Ansprüche 1 bis 4, **dadurch gekennzeichnet, dass** die Video-Kamera schrittweise verstellt wird.

6. Verfahren nach einem der Ansprüche 1 bis 5, **dadurch gekennzeichnet, dass** durch Scharfeinstellung mit Hilfe einer Autofokus-Funktion der Video-Kamera der Abstand zum beleuchteten Feld ermittelt wird und anschließend mittels der Steuereinheit Signale an das Stellglied der Kamera zum Ausgleich einer Parallaxe zwischen der optischen Achse der Kamera und des bzw. der Lichtbündel ausgegeben werden.

7. Verfahren nach Anspruch 6, **dadurch gekennzeichnet, dass** mit Hilfe einer Zoom-Optik eine maximale Vergrößerung des beleuchteten Gegenstandes erfolgt, bevor die Autofokus-Funktion der Video-Kamera aktiv wird.

8. Verfahren nach einem der Ansprüche 1 bis 7, **dadurch gekennzeichnet, dass** eine Korrektur der Ausrichtung der optischen Achse der Kamera auf iterativem Wege erfolgt.

9. Leuchte, insbesondere Operationsleuchte, mit wenigstens einem Leuchtgehäuse aus dem wenigstens ein Lichtbündel zur Bestrahlung eines Feldes in Richtung einer vorgegebenen Achse austritt, wobei das Leuchtungsgehäuse wenigstens eine Video-Kamera aufweist, die im Abstand zum Austrittsbereich des oder der Lichtbündel bzw. der vorgegebenen Achse angeordnet ist,
**dadurch gekennzeichnet, dass** die Video-Kamera (11) mit ihrer optischen Achse (13) gegenüber dem wenigstens einen Lichtbündel (5) bzw. gegenüber der vorgegebenen Achse des Leuchten-Gehäuses (1) mittels eines Stellgliedes einer Steuervorrichtung selbsttätig so weit verstellbar ist, dass die optische Achse (13) das beleuchtete Feld (8) schneidet.

10. Leuchte nach Anspruch 9, **dadurch gekennzeichnet, dass** zur Ansteuerung des Stellgliedes der Video-Kamera (11) eine Steuer-Einheit vorgesehen ist, welche Führungs-Signale erhält, die von der Ausrichtung der Video-Kamera in Bezug auf die vorgegebene Achse des Leuchten-Gehäuses (1) bzw. des Lichtbündels (5) bzw. der Lichtbündel abhängig ist.

11. Leuchte nach Anspruch 10, **dadurch gekenn-**
**zeichnet, dass** zur Bildung des jeweiligen Führungs-Signals ein Winkel-Sensor vorgesehen ist, der eine räumliche Ausrichtung der optischen Achse und des Leuchtungsgehäuses oder der vorgebenen Achse bzw. des Lichtbündels (5) erfasst.

12. Leuchte nach einem der Ansprüche 9 bis 11, **dadurch gekennzeichnet, dass** zur Bildung eines Führungs-Signals für die Steuereinheit ein Foto-Sensor vorgesehen ist.

Fig. 1

Fig. 2

EP 1 217 292 A1

## EUROPÄISCHER RECHERCHENBERICHT

Europäisches
Patentamt

Nummer der Anmeldung

EP 01 12 6069

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | US 5 347 431 A (BLACKWELL RAY A ET AL) 13. September 1994 (1994-09-13) * Spalte 2, Zeile 24 - Spalte 3, Zeile 26 * * Spalte 3, Zeile 56 - Spalte 4, Zeile 66 * --- | 1,9 | F21V33/00 H04N5/225 |
| D,A | DE 195 23 377 C (HERAEUS MED GMBH) 14. August 1996 (1996-08-14) * Spalte 2, Zeile 42 - Spalte 3, Zeile 38 * --- | 1,9 | |
| A | US 4 881 128 A (YAMADA MASAHIRO) 14. November 1989 (1989-11-14) --- | | |
| A | US 4 639 838 A (KATO YASUHIRO ET AL) 27. Januar 1987 (1987-01-27) ----- | | |

RECHERCHIERTE
SACHGEBIETE (Int.Cl.7)

H04N
F21V
A61B
F21S

Der vorliegende Recherchenbericht wurde für alle Patentansprüche erstellt

| Recherchenort | Abschlußdatum der Recherche | Prüfer |
|---|---|---|
| DEN HAAG | 26. März 2002 | Wentzel, J |

**ANHANG ZUM EUROPÄISCHEN RECHERCHENBERICHT**
**ÜBER DIE EUROPÄISCHE PATENTANMELDUNG NR.**          EP 01 12 6069

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-03-2002

| Im Recherchenbericht angeführtes Patentdokument | Datum der Veröffentlichung | Mitglied(er) der Patentfamilie | Datum der Veröffentlichung |
|---|---|---|---|
| US 5347431 A | 13-09-1994 | KEINE | |
| DE 19523377 C | 14-08-1996 | DE 19523377 C1<br>DE 59506726 D1<br>EP 0736725 A1<br>US 5808680 A<br>DE 29510724 U1 | 14-08-1996<br>07-10-1999<br>09-10-1996<br>15-09-1998<br>31-08-1995 |
| US 4881128 A | 14-11-1989 | KEINE | |
| US 4639838 A | 27-01-1987 | JP 61226031 A<br>KR 9004459 B1 | 07-10-1986<br>28-06-1990 |

Für nähere Einzelheiten zu diesem Anhang : siehe Amtsblatt des Europäischen Patentamts, Nr.12/82